# EUROPEAN PATENT APPLICATION

(11) **EP 1 884 233 A1**
(43) Date of publication of application: **06.02.2008**
(21) Application number: 06756507.7
(22) Date of filing: 24.05.2006
(51) Int. Cl.: A61K 9/44, B41M 5/26

(54) **METHOD OF MARKING COMPOSITION FOR ORAL ADMINISTRATION**

(30) Priority: 26.05.2005 JP 2005154745
(71) Applicant: Eisai R&D Management Co., Ltd., Tokyo 112-8088 (JP)
(72) Inventor: MOMOI, Kazuhisa, Kakamigahara-shi, Gifu 5016195 (JP)
(74) Representative: HOFFMANN EITLE
(86) International application number: PCT/JP2006/310301
(87) International publication number: WO 2006/126561

(57) **Abstract**

There is provided a marking method that is highly productive and enables production of easily-identifiable compositions for use in oral administration such as drugs and foods without damaging the quality of the oral composition. The marking method according to the present invention is a method for marking such a composition for use in oral administration and includes the steps of: dispersing a change in color-inducing oxide in the composition for use in oral administration; and scanning a surface of the composition for use in oral administration with a laser beam at wavelengths of from 200 nm to 1100 nm and with from 0.1 W to 50 W average power in to make the particles of the change in color-inducing oxide agglomerate so as to become discolored. The change in color-inducing oxide used in the present invention is at least one selected from the group consisting of titanium dioxide, yellow ferric oxide, and red ferric oxide.

## Description

### Technical Field

The present invention relates to a method for making an identification mark on a surface of compositions for use in oral administration such as drugs or foods.

### Background Art

With regard to pharmaceutical compositions for use in oral administration such as tablets and capsules, not only their packages, but also the compositions themselves are required to be identifiable in order to prevent mistakenly dispensing or taking the wrong drugs. However, there are limitations to identifying tablets and capsules with only their shapes and color tones. Therefore, marks are made on the surface of tablets and capsules in order to enhance identifiability. For example, the following methods are employed for marking tablets and capsules: transfer-type printing using a rubber roller to directly print letters and the like on a the surface of film-coated tablets and capsules; and inkjet-type printing of printing ink dots on the surface of film-coated tablets or capsules. In the case of uncoated tablets, a method for imprinting tablets, using a tableting punch with a concave-convex surface, has been employed since old times.

However, these classic methods like the transfer-type printing are easily affected by, for example, the surface condition of tablets or capsules and the atmosphere where the printing is carried out. Accordingly, very complicated management is required in order to stably provide clear printing. Also, in the case of inkjet-type printing, insufficient drying of ink causes stains, or blurring of the printed letters on the tablets, which may have an influence on the appearance of the tablets. Furthermore, in the case of imprinting, tablets and capsules have spherical surfaces in many cases and, therefore, there are limitations on the area on the tablet surface where letters and the like can be printed, and the number and size of letters or the shapes of graphic symbols that can be printed on the tablet surface. Consequently, the amount of information provided by the marked tablets is not always sufficient. Also, imprinting requires an exclusive punch for each product. Moreover, depending on the shape of the engraved mark, sticking may easily occur, resulting the engraved part being chipped off; and the engraved part may also wear off in the post-tableting step as well, which will cause an increase in the defect rate in an appearance examination. The engraved part may also chip off in the distribution process, which may not only decrease identifiability, but also have an influence on the product quality.

Therefore, as a method to replace the conventional printing methods and imprinting method, Patent Document 1, for example, discloses a method for marking tablets and capsules by irradiating them via a pattern mask with a laser beam of low intensity. Also, Patent Document 2, for example, discloses an etching method for evaporating, with laser, part of a tablet surface that can rapidly disintegrate in water; and a method of applying inkjet-type printing to the etched part of the tablet.
Patent Document 1: JP-A-4-122688
Patent Document 2: JP-T-2000-512303

### Disclosure of Invention

### Problems to be Solved by the Invention

However, in cases like Patent Document 1, where a pattern mask is used to make shadows, a contrast between the edges of the pattern mask, i.e., the boundary between the laser irradiated part and the non-irradiated part tends to become ambiguous, particularly on tablets and capsules with spherical surfaces where marks are made. Also, the pattern mask needs to be processed for each product in advance, and high precision processing techniques are necessary to process the pattern mask. Furthermore, since there are limitations on the shape and size of the mark, a sufficient amount of information cannot be provided by marking tablets and capsules.

In cases like Patent Document 2, where the laser beam is used to engrave the tablet surface, this method may damage the original properties of film-coated tablets and sugar-coated tablets, such as their moisture-proofedness, protection from light, and bitter taste masking. In the case of uncoated tablets, there are concerns that the drug content may be reduced due to etching, and that the etched part may cause part of the tablets to be chipped off. Moreover, since the etched part does not always clearly show the mark, it is sometimes necessary to further print the mark in ink. As a result, the same problems as that occur in the classic printing methods may occur.

Therefore, there is a strong need for development of a marking method unlike the conventional marking methods, which is highly productive and which enables production of easily-identifiable compositions for use in oral administration such as drugs and foods without damaging the quality of the compositions for use in oral administration.

### Means for Solving the Problems

As a result of thorough research of a method for marking the compositions for use in oral administration in light of the circumstances described above, the inventor of the present invention have found that irradiation of the surface of a composition for use in oral administration, in which a specified change in color-inducing oxide is dispersed, with a specified laser beam causes change in color of the surface due to agglomeration of the change in color-inducing oxide. As a result of this agglomeration phenomenon, the present inventor has completed the present invention.

According to a first aspect of the present invention, there is provided:
[1] A method for marking a composition for use in oral administration, comprising the steps of: dispersing a change in color-inducing oxide in the composition for use in oral administration; and
   scanning a surface of the composition for use in oral administration with a laser beam at wavelengths of from 200 nm to 1100 nm and with from 0.1 W to 50 W average power to make a particle of the change in color-inducing oxide agglomerate so as to become discolored;
[2] the marking method according to item [1], wherein the scanning step is carried out at from 20 mm/sec to 20000 mm/sec;
[3] the marking method according to item [1] or [2], wherein the scanning step is carried out with an energy per unit area of from 390 mJ/cm² to 21000 mJ/cm²;
[4] the marking method according to any one of items [1] to [3], wherein the laser beam is at least one beam selected from the group consisting of wavelengths of a solid state laser, and wavelengths of second, third, and fourth harmonic waves of the solid laser;
[5] the marking method according to any one of items [1] to [4], wherein the change in color-inducing oxide is at least one selected from the group consisting of titanium dioxide, yellow ferric oxide, and red ferric oxide;
[6] the marking method according to any one of items [1] to [5], wherein the composition for use in oral administration has a hardness of from 10 N to 500 N;
[7] the marking method according to any one of items [1] to [6], wherein the composition for use in oral administration is a molded product;
[8] the marking method according to any one of items [1] to [6], wherein the composition for use in oral administration comprises a coating layer;
[9] the marking method according to item [8], wherein the composition comprising the coating layer is a film-coated tablet;
[10] the marking method according to any one of items [7] to [9], wherein an amount of the titanium dioxide is from 0.01 to 20 parts by weight based on 100 parts by weight of the molded product or the coating layer;
[11] the marking method according to any one of items [7] to [9], wherein an amount of the yellow ferric oxide or the red ferric oxide is from 0.001 to 5 parts by weight based on 100 parts by weight of the molded product or the coating layer.

According to a second aspect of present the invention, there is provided:
[12] a method for manufacturing a marked composition for use in oral administration, comprising the steps of:
   dispersing a change in color-inducing oxide in a composition for use in oral administration; and
   scanning a surface of the composition for use in oral administration with a laser beam at wavelengths of from 200 nm to 1100 nm and with from 0.1 W to 50 W average power to make a particle of the change in color-inducing oxide agglomerate so as to become discolored;
[13] the manufacturing method according to item [12], wherein the scanning step is carried out at from 20 mm/sec to 20000 mm/sec;
[14] the manufacturing method according to item [12] or [13], wherein the scanning step is carried out with an energy per unit area of from 390 mJ/cm ² to 21000 mJ/cm ²;
[15] the manufacturing method according to any one of items [12] to [14], wherein the laser beam is at least one beam selected from the group consisting of wavelengths of a solid state laser, and wavelengths of second, third, and fourth harmonic waves of the solid laser;
[16] the manufacturing method according to any one of items [12] to [15], wherein the change in color-inducing oxide is at least one selected from the group consisting of titanium dioxide, yellow ferric oxide, and red ferric oxide;
[17] the manufacturing method according to any one of items [12] to [16], wherein the composition for use in oral administration has a hardness of from 10 N to 500 N;
[18] the manufacturing method according to any one of items [12] to [17], wherein the composition for use in oral administration is a molded product;
[19] the manufacturing method according to any one of items [11] to[17], wherein the composition for use in oral administration comprises a coating layer;
[20] the manufacturing method according to item [19], wherein the composition comprising the coating layer is a film-coated tablet;
[21] the manufacturing method according to any one of items [18] to [20], wherein an amount of the titanium dioxide is from 0.01 to 20 parts by weight based on 100 parts by weight of the molded product or the coating layer;
[22] the manufacturing method according to any one of items [18] to [20], wherein the amount of the yellow ferric oxide or the red ferric oxide is from 0.001 to 5 parts by weight based on 100 parts by weight of the molded product or the coating layer.

Moreover, according to a third aspect of the present invention, there is provided:
[23] a marked composition for oral composition manufactured by the manufacturing method according to any one of items 12 to 22;
[24] the oral composition according to item 23, wherein the composition for use in oral administration is a tablet or a capsule.

Furthermore, the present invention provides:
(1) a method for making an identification mark on a composition for use in oral administration comprising the steps of: dispersing titanium dioxide and/or yellow ferric oxide in the composition for use in oral administration; and scanning the composition for use in oral administration with a laser beam at wavelengths of from 200 nm to 1100 nm and with from 0.1 W to 50 W average power;
(2) the identification mark making method according to item (1), wherein the composition for use in oral administration is a molded product;
(3) the identification mark making method according to item (1), wherein the composition for use in oral administration has a coating layer;
(4) the identification mark making method according to item (2) or (3), wherein an amount of the titanium dioxide is from 0.01 to 10 parts by weight based on 100 parts by weight of the molded product or the coating layer;
(5) the identification mark making method according to item (2) or (3), wherein an amount of the yellow ferric oxide is 0.01 to 5 parts by weight based on 100 parts by weight of the molded product or the coating layer;
(6) a method for manufacturing a composition for use in oral administration, comprising the steps of: dispersing titanium dioxide and/or yellow ferric oxide in the composition for use in oral administration; and scanning the composition for use in oral administration with a laser beam at wavelengths of from 200 nm to 1100 nm and with from 0.1 W to 50 W average power to make an identification mark on the composition for use in oral administration;
(7) the manufacturing method according to item (6), wherein the composition for use in oral administration is a molded product;
(8) the manufacturing method according to item (6), wherein the composition for use in oral administration has a coating layer;
(9) the manufacturing method according to item (7) or (8), wherein an amount of the titanium dioxide is from 0.01 to 10 parts by weight based on 100 parts by weight of the molded product or the coating layer;
(10) the manufacturing method according to item (7) or (8), wherein an amount of the yellow ferric oxide is from 0.01 to 5 parts by weight based on 100 parts by weight of the molded product or the coating layer;
(11) a composition for use in oral administration with an identification mark made by the manufacturing method according to any one of items (6) to (10); and
(12) the composition for use in oral administration according to item (11), wherein the oral composition is a tablet or a capsule.

In a preferred embodiment of the manufacturing method according to the present invention, there is provided a method for manufacturing a composition for use in oral administration, including the steps of: obtaining a composition for use in oral administration, which has a coating layer in which at least one type of change in color-inducing oxide selected from the group consisting of titanium dioxide, yellow ferric oxide, and red ferric oxide, is dispersed; and making an identification mark by scanning a surface of the coating layer with a laser beam at wavelengths of from 200 nm to 1100 nm and with from 0.1 W to 50 W average power. The coating layer may comprise any of titanium dioxide, yellow ferric oxide, and red ferric oxide, but there is no particular limitation on combinations of these oxides or any other components. Specific examples of the coating layer include capsule coating layers, film-coated layers, and sugar-coated layers. The dosage forms of the composition for use in oral administration in a preferred embodiment of the present invention are: film-coated tablets, sugar-coated tablets, hard capsules, and soft capsules. Press coated tablets and the like whose outside layer is the coating layer may also be used.

In another preferred embodiment of the manufacturing method according to the present invention, there is also provided a method for manufacturing a composition for use in oral administration, including the steps of: obtaining a molded product in which at least one type of change in color-inducing oxide selected from the group consisting of titanium dioxide, yellow ferric oxide, and red ferric oxide, is dispersed; and making an identification mark by scanning a surface of the molded product with a laser beam at wavelengths of from 200 nm to 1100 nm and with from 0.1 W to 50 W average power. Specific examples of the molded product include: compositions for use in oral administration in the form of uncoated tablets, orally rapid disintegrating tablets, troches, press coated tablets, and the like. These dosage forms can be used not only for drugs, but also for foods. Moreover, a composition for use in oral administration, which has a coating layer, can be prepared by making an identification mark on the molded product and then applying a transparent film coating over the molded product.

Note that the term "change in color-inducing oxide" used in the present invention means an oxide whose a particle agglomerate and is thereby induced to become discolored due to laser beam irradiation.

### Advantageous Effects of the Invention

According to the present invention, the laser-irradiated titanium dioxide, yellow ferric oxide, and red ferric oxide undergo no chemical change such as decomposition, provide a high level of safety, and can be manufactured in a clean condition without requiring any special solvent. Therefore, the manufacturing method according to the present invention can be used to manufacture compositions for use in oral administration such as foods and drugs. Also, according to the present invention, the compositions for use in oral administration such as foods and drugs can be manufactured in normal manufacturing lines without reducing productivity.

Moreover, according to the present invention, a clear mark can be made at any position on the composition according to the present invention and hardly be affected by the shape and roughness of the surface of the composition by dispersing, in the composition for use in oral administration, at least one type of change in color-inducing oxide selected from the group consisting of titanium dioxide, yellow ferric oxide, and red ferric oxide. Accordingly, the present invention can preferably be applied to the compositions for use in oral administration, which has spherical surfaces such as tablets and capsules, as well as foods with non-level surfaces such as confectionery. In addition, since the tablet surface is not etched very much, it is suitable to provide a method for manufacturing film-coated tablets and capsules with a coating layer, and enhance merchantability and quality.

Furthermore, since the laser beam can be controlled with precision by, for example, a galvanometer mirror, the present invention provides greater flexibility to the design of an identifiable mark and the design can be marked on the composition with ±10µm precision. Accordingly, it is possible to provide a method for making an identification mark on a composition for use in oral administration, which has a complicated design or a composition for use in oral administration with a diameter of 2 to 3 mm, like minitablets. Also, since information about the mark design is input as a signal from a computer or the like, it is easy to change the design.

### Brief Description of Drawings

Fig. 1 illustrates a schematic diagram of an apparatus for implementing the marking method according to the present invention;
Fig. 2 illustrates a flow chart showing a method for manufacturing the composition for use in oral administration used in the present invention;
Fig. 3 shows a TEM photograph of a molded product containing titanium dioxide according to the present invention prior to laser irradiation; and
Fig. 4 shows a TEM photograph of the molded product containing titanium dioxide according to the present invention after laser irradiation.

### Best Mode for Carrying Out the Invention

The embodiments described below are for the purpose of describing the present invention, but the present invention is not limited only to these embodiments. Accordingly, the present invention can be utilized in various ways without departing from the gist and the spirit of the present invention.

### (Marking Method)

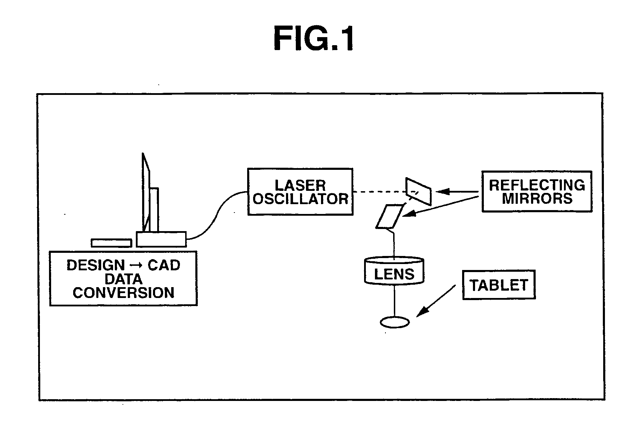

Fig. 1 illustrates a schematic diagram of an apparatus for implementing the marking method according to the present invention. Although Fig. 1 shows one example of the apparatus for implementing the marking method according to the present invention, the apparatus is not limited to the one shown in Fig. 1. If CAD design information is input to a controller, that information is given to a laser oscillator and galvanometer mirrors. Subsequently, a laser beam is emitted from the oscillator, collected by the galvanometer mirrors or the like, and then applied to the target, the surface of a composition for use in oral administration. At least one type of the change in color-inducing oxide selected from the group consisting of titanium dioxide, yellow ferric oxide, and red ferric oxide is dispersed in the composition for use in oral administration used in the present invention. It was found that the laser irradiation according to the present invention makes the dispersed change in color-inducing oxide such as titanium dioxide partly agglomerate and become discolored. Such an effect cannot be obtained by any other metallic oxides if used in the composition according to the present invention. An identifiable mark can be made on the composition for use in oral administration by the marking method according to the present invention, without affecting any other components of the composition for use in oral administration (described later) or deteriorating the appearance of the composition. Since the information input to the controller can be changed immediately with the marking method according to the present invention while observing the irradiation state, it is easy to modify the design.

### (Laser)

There are no particular limitations on the type of laser used in the marking method according to the present invention, and lasers capable of laser oscillation with a laser medium (source of laser oscillation) in a solid, liquid, or gas state respectively can be used. However, a solid state laser can preferably be used. Examples of the solid state laser include YLF lasers, YAG lasers, and YVO4 lasers. In particular, basic, second, third, fourth, and fifth harmonic YVO4 laser oscillators can preferably be used. Specifically, Prisma Series Lasers (by Coherent Inc., Model Number: 532-12-V) can be used as the second harmonic oscillator, a YVO4 Laser DS20H-355 (by Photonics Industries International, Inc) as a third harmonic oscillator, and AVIA266-3000 (by Coherent Inc.) as a fourth harmonic oscillator.

### (Laser Wavelength)

Lasers with wavelengths of from 200 to 1100 nm can be used as the laser for use in the present invention, preferably with wavelengths of from 1060 to 1064 nm, from 527 to 532 nm, from 351 to 355 nm, from 263 to 266 nm, or from 210 to 216 nm, and more preferably with wavelengths of from 527 to 532 nm, from 351 to 355 nm, or from 263 to 266 nm.

### (Laser Output)

The average power for laser scanning in the present invention can be within a range that hardly etches the surface of the target composition for use in oral administration. The average power is, for example, from 0.1 W to 50 W, preferably from 1 W to 35 W, and more preferably from 5 W to 25 W. If the laser irradiation energy per unit time is too strong, the tablet surface will be etched due to ablation and the discolored part will chip away. On the other hand, if the output is weak, change in color will not be sufficient.

### (Laser Scanning Method)

There are no particular limitations on the laser scanning method for irradiating the surface of the composition for use in oral administration with a laser. Examples of the laser scanning method include: a galvanometer mirror method using a deflector capable of changing the rotation angle of a mirror according to an electric signal; a resonant scan method using a resonant deflector that is driven by sine waves of resonance frequencies; and a polygon mirror method using a plurality of mirrors or the like. However, the galvanometer mirror method should preferably be used. There are no particular limitations on the scanning speed, being from 20 mm/sec to 20000 mm/sec, preferably from 60 mm/sec to 8000 mm/sec and more preferably from 80 mm/sec to 8000mm/sec. If the laser output is 0.3 W, the lower limit of the scanning speed is 20 mm/sec or more. If the scanning speed is 8mm/sec, etching may occur; and if the scanning speed is 2mm/sec, the laser irradiation part may sometimes be burned. If the laser output is 5 W, the lower limit of the scanning speed is 80 mm/sec or more, preferably 100 mm/sec or more. If the scanning speed is 60 mm/sec or less, etching may occur or the laser irradiation part may sometimes be burned. Higher scanning speeds can increase productivity without affecting the identifiability of the mark. Therefore, if the laser output is 5 W, the scanning speed is from 80 mm/sec to 10000 mm/sec, preferably from 90 mm/sec to 10000 mm/sec and more preferably from 100 mm/sec to 10000 mm/sec. If the laser output is 8 W, the scanning speed is from 250 mm/sec to 20000 mm/sec, preferably from 500 mm/sec to 15000 mm/sec and more preferably from 1000 mm/sec to 10000mm/sec.

### (Energy per Unit Area)

Laser energy per unit area for irradiating and scanning the surface of the composition for use in oral administration with a laser according to the present invention is from 390 to 21000 mJ/cm², preferably from 400 to 20000 mJ/cm² and more preferably from 450 to 18000 mJ/cm², from the viewpoint of whether or not marking can be made and whether or not etching would be caused. If the energy per unit area is lower than 390 mJ/cm², marking cannot be made. If the energy per unit area is higher than 21000 mJ/cm², etching will be caused, which is a problem. The compositions for use in oral administration such as rapidly disintegrating tablets or the compositions for use in oral administration, which has an enteric coating layer, should preferably be irradiated with a laser at an energy level within the range described above.

### (Manufacturing Method)

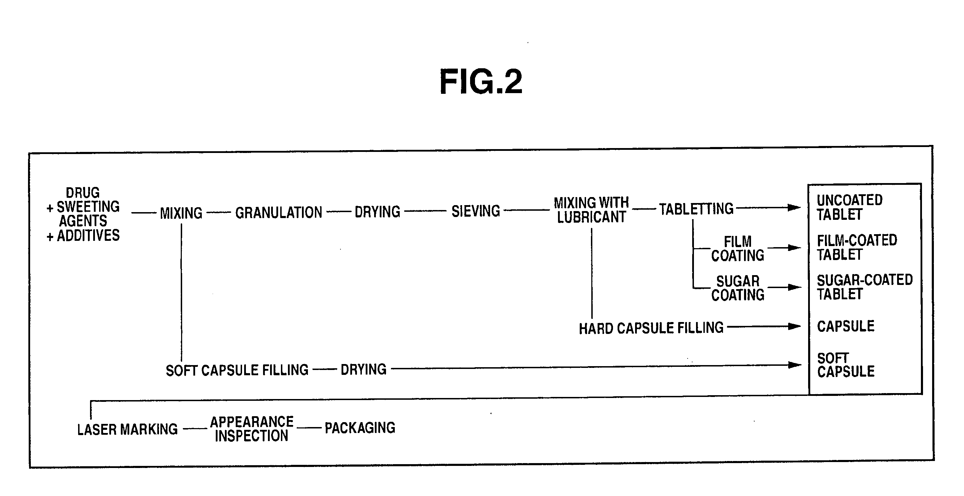

Fig. 2 illustrates a flowchart showing a method for manufacturing the composition for use in oral administration used in the present invention. Note that Fig. 2 illustrates an example of the manufacturing method according to the present invention, by using a pharmaceutical composition for use in oral administration. However, the manufacturing method according to the present invention is not limited to the use of the pharmaceutical composition for use in oral administration. For example, in the case of uncoated tablets, all the components are mixed, the mixture is granulated as necessary, the obtained mixture is compressed and molded into molded products, and then the molded products are irradiated with a laser. Subsequently, the composition for use in oral administration undergoes examinations by, for example, a letter test machine and a metal test machine, and then can be either discharged to a conveying drum or filled in packages. Accordingly, there is no particular limitation on the laser irradiation timing as long as the laser irradiation is carried out after at least one type of the change in color-inducing oxide selected from the group consisting of titanium dioxide, yellow ferric oxide, and red ferric oxide is added to the composition for use in oral administration. Therefore, the laser irradiation process can be freely incorporated into the steps of manufacturing foods and drugs. For example, tablets carried on a conveyer line before filling packages can be irradiated with a laser and, therefore, the laser irradiation process can be incorporated into so-called continuous production. Also, tablets may be automatically spread on a flat pan, on which a certain amount of tablets can be placed, in the middle of the conveyer line, and then may be made to stand still and be irradiated with a laser. Alternatively, an automatic marking device having a laser oscillator can be utilized to perform batch processing. Specifically, tablets or the like filled in the marking device tablet hopper are sequentially supplied from the hopper to an inspection department. Subsequently, an appearance inspection machine is used at the inspection department to check whether or not there are any stains, broken parts, or cracks on both sides of the tablets. Then, one or more tablets are moved at one time to a laser irradiation position and can be marked with a laser.

### (Mark)

The mark according to the present invention should preferably be an identifiable mark. The mark is indicated on drugs in order to ensure identifiability and differentiate them from other drugs according to the design of the mark. However, there is no particular limitation on the mark according to the present invention, and a user can freely select any mark according to design requirements. A mark made by the marking method according to the present invention is characterized by its higher degree of design freedom as compared to conventional methods. There is no particular limitation on mark designs, and letters, numbers, graphics, and symbols can be used singly or in combination. It is possible to use, for example, product names, abbreviated product names, sales company marks or logos, or various codes used for drugs, such as their Japan Standard Commodity Classification Number, Therapeutic Category Number, National Health Insurance Drug Code, and Universal Product Code or the like.

### (Change in color-inducing Oxide)

The change in color-inducing oxides used in the present invention are metal oxides used for marking. At least one type of change in color-inducing oxide selected from the group consisting of titanium dioxide, yellow iron ferric oxide, and red ferric oxide can be used. The type of titanium dioxide used in the present invention is not particularly limited, and includes rutile type, anatase type or a combination of these types, or titanium dioxide processed to contain silica or the like, or titanium oxide modified by silica or the like. For example, products listed in the Japanese Pharmacopoeia such as "titanium dioxide" (Product Name by Kawazu Sangyo Co., Ltd.) can be used.

The yellow ferric oxide used in the present invention is a natural pigment that is listed in the Japanese Pharmaceutical Excipients 2003 ("JPE") and used as a coloring agent. As the yellow ferric oxide, for example, "yellow ferric oxide Colorcon" (Product Name by Colorcon (Japan) Limited) and "yellow ferric oxide" (Product Name by JUNSEI CHEMICAL CO., LTD) can be used. The yellow ferric oxide is a yellow to brownish-yellow powder and is practically insoluble in water.

The red ferric oxide used in the present invention is a natural pigment that is listed in the JPE and used as a coloring agent. For example, "red ferric oxide" (Product Name by JUNSEI CHEMICAL CO., LTD) can be used. The red ferric oxide is a red to reddish-brown or dark magenta powder and is practically insoluble in water.

It is preferable that the change in color-inducing oxides used in the present invention be dispersed in a surface of the composition for use in oral administration, regardless of dosage forms of the composition for use in oral administration.

The amount of titanium dioxide used in the present invention is not particularly limited, and is, for example, from 0.01 to 20 parts by weight, preferably from 0.05 to 15 parts by weight, more preferably from 0.5 to 10 parts by weight, and most preferably from 2 to 4 parts by weight, based on 100 parts by weight of the molded product or the coating layer. The amount of yellow ferric oxide used in the present invention is not particularly limited, and is, for example, from 0.001 to 5 parts by weight, preferably from 0.01 to 2 parts by weight, more preferably from 0.05 to 1 part by weight, and most preferably from 0.1 to 0.5 parts by weight, based on 100 parts by weight of the molded product or the coating layer. The amount of red ferric oxide used in the present invention is not particularly limited, and is, for example, from 0.001 to 5 parts by weight, preferably from 0.01 to 2 parts by weight, more preferably from 0.05 to 1 part by weight, and most preferably from 0.1 to 0.5 parts by weight, based on 100 parts by weight of the molded product or the coating layer. These components are evenly dispersed and exist at least in the area to be marked in the coating layer or the molded product prior to the laser irradiation.

There is no particular limitation on the target to be marked with a laser in the laser marking method according to the present invention, as long as it is a composition for use in oral administration. However, the hardness of the composition for oral administration is preferably from 10 to 500 N, more preferably from 20 to 400 N and most preferably from 20 to 300 N. The hardness of the target can be measured, using a Kiya-type hardness tester (by FUJIWARA SCIENTIFIC CO., LTD; 043019-E).

### (Molded Product)

The molded product as one aspect of the composition for use in oral administration used in the present invention is formed by using a mixture containing at least one type of the change in color-inducing oxide selected from the group consisting of titanium dioxide, yellow ferric oxide, and red ferric oxide, together with the drugs and sweeting agents or other additives described later. There is no particular limitation on the molded product as long as it is not powder and has a certain shape. The molded product can be formed by, for example, the following processes: obtaining a mixture in a powder or granular form containing the drugs by using a V-type mixing machine (by TOKUJU CORPORATION, Fuji Paudal Co., Ltd., or DALTON CORPORATION), a tumbler mixing machine (by DALTON CORPORATION or DAIKO SEIKI CO., LTD.), or a high-speed agitating mixer (by OKADA SEIKO CO., LTD., NARA MACHINERY CO., LTD., or Powrex Corp.); then either compressing and molding the obtained mixture by using a tablet making machine such as a single punch tableting machine, a rotary tableting machine, or a dry coated tablet making machine, or by using a molding machine and charging a mold with suspension or slurry in which the mixture is dispersed in water or some other solvent, and drying the solvent. Specifically, examples of the molded product as one aspect of the composition for use in oral administration used in the present invention include uncoated tablets, orally rapidly disintegrating tablets, troches, chewable tablets, pills or the like, which can be easily recognized by those skilled in the art, but the molded product is not limited to these examples. Other examples of the molded product as another form of the composition for use in oral administration used in the present invention include foods such as lemon kali, gum, candies, and mouth freshening foods that can be easily recognized by those skilled in the art, but the molded product is not limited to these examples. A transparent coating layer that would give no damage to the identifiability of a mark may be provided on the molded products described above after making the identifiable mark on the molded products.

### (Coating Layer)

In the coating layer as one aspect of the composition for use in oral administration used in the present invention, at least one type of the change in color-inducing oxide selected from the group consisting of titanium dioxide, yellow ferric oxide, and red ferric oxide is evenly dispersed at least in the marking area. Moreover, the position of the coating layer as one aspect of the composition for use in oral administration used in the present invention is not particularly limited, as long as the coating layer is located in the area that can be reached by a laser beam energy during laser irradiation. However, the coating layer should preferably be positioned as the outermost layer of the composition for use in oral administration for the laser irradiation. Examples of the coating layer include a capsule coating layer, a film-coated layer, a sugar-coated layer or the like. It is also possible to coat the marked composition for use in oral administration with a layer transparent enough to give no damage to the identifiability of the mark. As a method for manufacturing the coating layer or the composition with the coating layer as an aspect of the composition for use in oral administration used in the present invention, known methods can be applied singly or in combination thereof. In the case of a capsule coating layer, the capsule coating layer can be manufactured by a soft capsule manufacturing method such as a rotating die method or a falling-drop method, and hard capsules can be manufactured in advance separately from the manufacture of the composition for use in oral administration. In the case of a film-coated layer and a sugar-coated layer, examples of the coating method include, but are not limited to, a coating granulation method, a film coating method, a sugar coating method, a compression coating method or the like.

### (Composition for use in Oral Administration with Coating Layer)

The composition for use in oral administration used in the present invention, which has a coating layer, should preferably be a composition that is made by coating a core containing drugs and sweeting agent components, with the coating layer in which at least one type of metal oxide selected from the group consisting of titanium dioxide, yellow ferric oxide, and red ferric oxide is dispersed, and having the coating layer marked on its surface. Examples of the dosage form include film-coated tablets, sugar-coated tablets, and pills; the film-coated tablets being preferably used. Needless to say, foods with the coating layer, such as chocolates and mouth fresheners may constitute the composition for use in oral administration. There is no particular limitation on the form of the core. For example, drugs and sweeting agent components can be evenly mixed with other agents such as diluents and binders as necessary, and then the obtained mixture can be granulated into granular form by wet granulation or dry granulation; or a nonpareils such as sugar cores or cellulose cores can be coated with a layer containing the drugs, thereby obtaining granules. These granules can further be compressed and molded into tablet-shaped cores. Alternatively, the granules can be compressed and molded into pill-shaped cores, which will be covered with a plurality of layers to become pills.

### (Capsules)

Capsules can also be an example of the composition for use in oral administration with the coating layer used in the present invention. Such capsules are made by filling a capsule coating layer, which contains at least one type of metal oxide selected from the group consisting of titanium dioxide, yellow ferric oxide, and red ferric oxide, with contents including drugs, food oil, flavors or the like, and then making an identifiable mark on the capsule coating layer. Such capsules are not limited to either hard capsules or soft capsules. The contents including the drugs may be used alone; or the contents may be mixed with other additives as necessary, and the mixture may be used in a powder or granular form, or as molded products formed from that powdery or granular mixture, or in a liquid or slurry state. For example, gelatin capsules, agar capsules, HPMC capsules, pullulan capsules, starch capsules or the like can be used as the capsule coating layer as long as they contain at least one type of metal oxide selected from the group consisting of titanium dioxide, yellow ferric oxide, and red ferric oxide. However, the capsule coating layer is not limited to the examples listed above. In order to fill the capsules, generally used filling machines such as a rotating die capsule filling machine, a seamless capsule filling machine, and a hard capsule filling machine (by Qualicaps Co., Ltd.) or the like can be used.

### (Drugs)

The drugs used in the present invention include active ingredients such as therapeutic drugs, diagnostic reagents, and quasi drugs, specified health foods, supplement foods or the like. The therapeutic drugs may be drugs that contain synthetic compounds, and drugs derived from biological origin such as proteins, peptides or the like. The diagnostic reagents are X-ray contrast mediums and other drugs used for diagnosis. There is no particular limitation on the types of the drugs used in the present invention, and examples of the drugs, but are not limited to, include antitumor drugs, antibiotics, anti-inflammatory drugs, analgesics, osteoporosis drugs, antihyperlipemic drugs, antibacterial drugs, tranquilizers, antiepileptic drugs, antidepressants, therapeutic drugs for alimentary disease, therapeutic drugs for allergic disease, therapeutic drugs for high blood pressure, therapeutic drugs for arteriosclerosis, and therapeutic drugs for diabetes, hormonal drugs, fat-soluble vitamin drugs, herbal medicines or the like. The active ingredients of quasi drugs and the like have mild effects, and examples of the active ingredients include amino acids, vitamins, polyphenols or the like. The drugs and active ingredients described above can be contained, whether only one kind or two or more kinds, in the composition for use in oral administration according to the present invention

### (Other Additives)

Other additives used in the present invention are pharmacologically acceptable substances. Specific examples of the additives include diluents, binders, lubricants, disintegrators, coating agents, plasticizers, suspending agents or emulsifiers, flavors, sweeting agents, sugar coating agent, fluidizing agents, colorants, solvents or the like. Specific examples of the diluents include, but are not limited to, D-mannitol, lactose, sucrose, corn starch, crystalline cellulose, anhydrous calcium hydrogenphosphate, precipitated calcium carbonate or the like. Specific examples of the binders include, but are not limited to, povidone, dextrin, hydroxypropylcellulose, hydroxypropylmethylcellulose, methylcellulose, polyvinyl alcohol, sodium carboxymethylcellulose, α-starch or the like. Specific examples of the lubricants include, but are not limited to, hydrogenated oil, stearic acid, magnesium stearate, stearyl sodium fumarate or the like. Specific examples of the disintegrators include, but are not limited to, low-substituted hydroxypropylcellulose, carmellose, sodium carboxymethyl starch, crosspovidone or the like. Specific examples of the coating agents include, but are not limited to, cellulose derivatives such as hydroxypropylcellulose, hydroxypropylmethylcellulose, carmellose sodium or the like; acrylic polymers such as dispersions of ethyl acrylate and methyl methacrylate copolymers, methacrylate copolymer-L or the like; synthetic polymer substances such as polyvinyl pyrrolidone, polyvinyl alcohol, polyoxyethylene polyoxypropyleneglycol or the like; and natural polymer substances such as gelatin and acacia gum or the like. Specific examples of the plasticizers include, but are not limited to, dioctyl adipate, triethyl citrate, triacetin, glycerin, concentrated glycerin, propyleneglycol or the like. Specific examples of the suspending agents or emulsifiers include, but are not limited to, lecithin, sucrose fatty ester, polyglycerol fatty ester, polyoxyethylene hydrogenated castor oil, polysorbate, polyoxyethylene-polyoxypropylene copolymers or the like. Specific examples of the flavors include, but are not limited to, menthol, peppermint oil, orange oil or the like. Specific examples of the sweeing agents include, but are not limited to, extracted materials from, or flavor components having flavors of, edible plants, fruits, seeds, and tea leaves. Specific examples of the sugar coating agents include, but are not limited to, sucrose, lactose, starch syrup, precipitated calcium carbonate, acacia gum, carnauba wax or the like. Specific examples of the fluidizing agents include, but are not limited to, hydrous silicon dioxide, light anhydrous silicic acid, calcium stearate, talc, corn starch or the like. Specific examples of the colorants include, but are not limited to, tar dyes such as Food Yellow No. 4, Food Yellow No. 5, Food Red No. 2, Food Red No. 102, Food Blue No. 1, Food Blue No. 2 (indigo carmine), Food Yellow No. 4, aluminum lake or the like; red ferric oxide (colcothar); talc; riboflavine; or the like. Specific examples of the solvent include, but are not limited to, water, ethyl alcohol, isopropyl alcohol, ethyl acetate, propylene glycol, peanut oil, castor oil, sesame oil, glycerin, polyethylene glycol or the like.

### Examples

The present invention will be described in further detail below by referring to examples and comparative examples, but the invention is not limited only to these examples and comparative examples. As additives in the pharmaceutical composition for use in oral administration, those compliant with official compendia such as Japanese Pharmacopoeia, Japanese Pharmaceutical Excipients 2003 (hereinafter referred to as "JPE"), Japanese Pharmaceutical Codex 1997 (hereinafter referred to as "JPC"), and Japanese Standards for Food Additives (hereinafter referred to as "JSFA"), or commercially available reagents were used. Those skilled in the art can put the invention into practice by making variations of the following examples, and any such variations will be within the scope of the claims of this present invention.

### (Example 1) Tablets

Tablets were prepared by mixing 80 g of lactose, 20 g of corn starch, 0.5 g of magnesium stearate, and 1 g of titanium dioxide, or 0.2 g of yellow ferric oxide, and compressing and molding the mixture, using a tablet making machine or an autograph material tester.

A laser (manufactured by Photonics Industries International, Inc.) shown in Table 1 was used to irradiate 100 tablets spread over a 10 cm × 10 cm pan with a laser under conditions described in Table 1. As a result, tablets with highly visible marks were obtained when they contained titanium dioxide or yellow ferric oxide. It was confirmed that their surfaces were not etched.

### (Example 2) Tablets

Tablets were prepared by mixing 80 g of lactose, 20 g of corn starch, 0.5 g of magnesium stearate, and 0.01 g of titanium dioxide or 0.006 g of yellow ferric oxide, and compressing and molding the mixture, using a tablet making machine or an autograph material tester. These tablets were marked, using the laser and under the irradiation conditions described in Table 1. As a result, change in color was observed and visible marks slightly inferior to those in Example 1 were obtained.
[Table 1]

**Table 1**

| Laser Name | YVO4 Laser |
|---|---|
| Model | DS20H-355 |
| Wavelength | 355nm |
| Average Power at 20kHz | 8W |
| Pulse Width at 20kHz | 25ns |
| Pulse Energy at 20kHz | 0.4mJ |
| Beam Mode | TEMₒₒ - M² < 1.1 |
| Polarization Ratio | 100:1 Horizontal |
| Beam Diameter | 0.9mm |
| Beam Divergence | 1.3mrad |
| Pulse-to-Pulse Stability | 3%rms |
| Long-Term Instability | +/-3% |
| Pointing Stability | < 50µrad |
| Pulse Repetition Rate | Single Shot to 100kHz |

| | |
|---|---|
| Manufacturer: Photonics Industries International, Inc. | |

### (Comparative Examples 1 to 10)

Uncoated tablets were prepared in the same manner as in Example 1, except that additives (0.2 g in each comparative example) were mixed instead of titanium dioxide and yellow ferric oxide. Then, the uncoated tablets were scanned with a laser beam under the same conditions as in Example 1.

The uncoated tablets containing the above-described components did not change in color, confirming that the additives used in Comparative Examples 1 to 10 are not effective as change in color-inducing agents for the compositions for use in oral administration.
[Table 2]

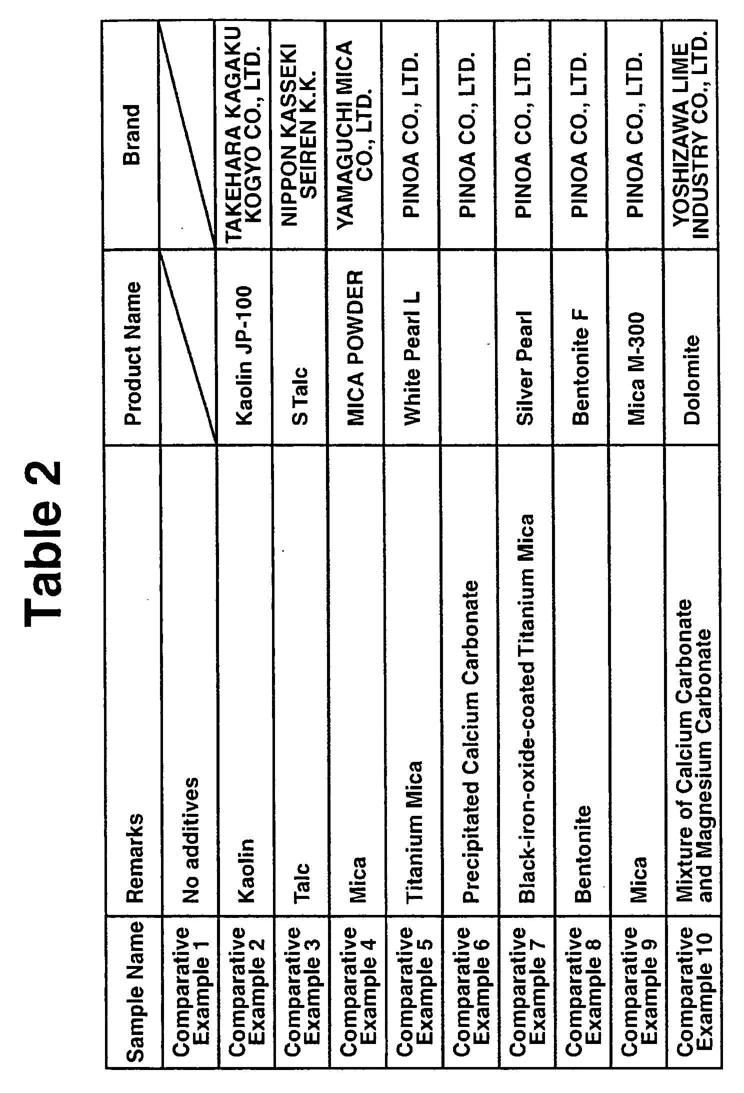

### (Example 3) Soft Capsules

Soft capsules having a gelatin coating layer that contains 20 parts by weight of concentrated glycerin, 10 parts by weight of D-sorbitol, and 1 part by weight of titanium dioxide based on 100 parts by weight of gelatin, were prepared using a rotating die method. These soft capsules (white color) were marked with letters "E268" along their longitudinal diameter direction, using the laser and under the irradiation conditions described in Table 1. As a result, soft capsules with highly visible gray marks were obtained. On the other hand, an attempt was made to mark the soft capsules with the same letters, using a carbon dioxide laser (manufactured by KEYENCE CORPORATION; ML-G9300 Series; 1060 nm). As a result, white letters were formed by a foaming phenomenon on the surface of the soft capsules, but the visibility of the white letters was slightly poor on the white base color of the capsules.

### (Example 4) Sugar-coated Tablets

Spindle-shaped sugar-coated tablets (7.15 mm in diameter and 3.95 mm thick) were prepared by using a sugar coater to coat each uncoated tablet with approximately 55 g of sugar coating layer (2 mg of precipitated calcium carbonate, 8 mg of talc, 4.8 mg of titanium dioxide, 1 mg of hydrous silicon dioxide, 0.4 mg of povidone, 1.6 mg of powdered acacia, 1.4 mg of corn starch, 0.2 mg of macrogol 6000, and 36 mg of sucrose). During the sugar coating, sampling was carried out at regular time intervals until the end of the sugar coating process. As a result, white sugar-coated tablets with coating layers of different thicknesses were obtained (eight kinds of samples including those with the complete sugar coating layer). These samples were marked with a center line passing the center point of each sugar-coated tablet and extending from one side of the tablet to the other, using the laser and the irradiation conditions described in Table 1. As a result, a dark gray center line was successfully marked on the tablets, irrespective of the thicknesses of the coating layers. No color tone difference was observed between the center area of each tablet and its edge area.

### (Example 5) Orally Rapid Disintegrating Tablet

Orally rapid disintegrating cylindrical tablets, that contain 0.18 parts by weight of yellow ferric oxide based on 100 parts by weight of tablet (principal component: D-mannitol [Japanese Pharmacopeia]) were marked with a center line passing the center point of each tablet and extending from one side of the tablet to the other, using the laser and the irradiation conditions described in Table 1. The tablets were also irradiated with a laser in the same manner as described above, using the laser and the irradiation conditions described in Table 3. As a result, a dark gray center line was successfully marked on the tablets in both cases. The center line of the tablets irradiated with the laser as described in Table 3 turned out to be a darker gray than the center line of the tablets irradiated with the laser described in Table 1. In either irradiation, no etching occurred on the tablet surfaces.
[Table 3]

**Table 3**

| Laser Name | YVO4 Laser |
|---|---|
| Model | AVIA266-3000 |
| Wavelength nm | 266 |
| Average Power W | > 3 at 30kHz |
| Pulse Repetition Rate kHz | Single Shot to 100kHz |
| Pulse Width ns | < 25 at 30kHz |
| Pulse-to-Pulse Stability %rms | < 5 at 30kHz |
| Polarization (Extinction Ratio) | Linear Horizontal (> 100 : 1) |
| Warm-up Time minutes | < 10 minutes |

| | |
|---|---|
| Manufacturer: Coherent, Inc.; High Power Pulse Deep-UV Laser | |

### (Examples 6 to 8)

According to the procedures of Example 5, orally rapid disintegrating cylindrical tablets, that contain 0.04 parts by weight (Example 6), 0.08 parts by weight (Example 7), or 0.18 parts by weight (Example 8) of yellow ferric oxide based on 100 parts by weight of tablet, were marked with a center line passing the center point of each tablet and extending from one side of the tablet to the other, using the laser and the irradiation conditions described in Table 4. As a result, yellow tablets with a gray mark were obtained with no etching on the tablet surfaces.
[Table 4]

**Table 4**

| Specification | |
|---|---|
| Model Name | 532-12-V |
| Wavelength nm | 532 |
| Excitation Medium | Nd :YVO₄ |
| Average Power W | - |
| | 12 (pulsed) |
| Pulse Energy (at 30kHz) | 400 |
| Pulse Width ns | 18 (at 30kHz) |
| Pulse Repetition Rate kHz | 20-100 |
| Peak Power (at 30kHz) kW | 22 |
| Pulse-to-Pulse Stability (at 30kHz) % | < 3.5 |
| Polarization (Extinction Ratio) | Linear Horizontal (100 : 1) |
| Space Mode | TEM₀₁ (M² < 2.4) |
| Beam Diameter (1/e²) mm | 1.2 |
| Beam Divergence (Whole Angle) mrad | 1.4 |

| | |
|---|---|
| Manufacturer: Coherent, Inc.; Prisma^{™} Series | |

### (Example 9)

Tablets were prepared by mixing 80 g of lactose, 20 g of corn starch, 0.5 g of magnesium stearate, and 0.006 g of titanium dioxide, and compressing and molding the mixture, using a tablet making machine or an autograph material tester. The tablets were irradiated with the laser and under the irradiation conditions described in Table 1. As a result, tablets with highly visible marks were obtained. It was confirmed that their surfaces were not etched.

### (Examples 10 and 11)

According to the procedures of Example 5, orally rapid disintegrating cylindrical tablets, that contain 0.025 parts by weight (Example 10) or 0.18 parts by weight (Example 11) of red ferric oxide based on 100 parts by weight of tablet, were marked with a center line passing the center point of each tablet and extending from one side of the tablet to the other, using the laser and the irradiation conditions described in Table 1. As a result, no etching was observed on the tablet surfaces, and red tablets with a light blue mark were obtained.

### (Comparative Example 11)

Orally rapid disintegrating tablets that contain the same components as those in the orally rapid disintegrating tablets in Example 5, except no yellow ferric oxide, titanium dioxide or red ferric oxide, were irradiated with a laser in the same manner as in Example 5. The tablets were neither marked nor etched.

### (Example 12) Film-coated Tablets

Film-coated tablets, each having curved surfaces and a cylindrical part, were prepared by coating each uncoated tablet with a coating layer (principal component: hydroxylpropylmethylcellulose terephthalate [Japanese Pharmacopeia]) that contained 3.4 parts by weight of titanium dioxide and 0.4 parts by weight of yellow ferric oxide based on 100 parts by weight of the coating layer, by using an automatic film coating machine. Each tablet was marked with eleven letters in a circular pattern around its curved area, using the laser and under the irradiation conditions described in Table 1. It was confirmed that all the letters were highly identifiable and even the peripheral edge area could be sufficiently marked.

### (Example 13) Film-coated Tablets

Film-coated tablets, each having curved surfaces and a cylindrical part, were prepared by coating each uncoated tablet with a coating layer (principal component: hydroxyl propylmethylcellulose terephthalate [Japanese Pharmacopeia]) that contained 4 parts by weight of titanium dioxide based on 100 parts by weight of the coating layer, by using an automatic film coating machine. Each tablet was marked with letters in three lines on its curved surface, using the laser and on the irradiation conditions as described in Table 1. Also, the tablets were marked in the same manner, using a YVO4 laser (manufactured by KEYENCE CORPORATION; MD-V9600) at a base wavelength of 1064 nm. As a result, the former laser succeeded in marking the tablets with the letters in dark gray. The latter laser resulted in marking the tablets with the letters in a slightly light gray, and it was confirmed that the identifiability of the letters in the latter case was slightly inferior to that of the letters in the former case.

### (Example 14)

According to the procedures of Examples 12 and 13, film-coated tablets, each having curved surfaces and a cylindrical part, were prepared by coating each uncoated tablet with a coating layer that contained 0.6 parts by weight of red ferric oxide and 12.9 parts by weight of titanium dioxide based on 100 parts by weight of the coating layer. Each tablet was marked with letters in three lines on its curved surface, using the laser and under the irradiation conditions described in Table 1. As a result, the tablets were successfully marked with the letters in dark gray.

### (Example 15) Sugar-coated Tablets

Spindle-shaped sugar-coated tablets (6.2 mm in diameter and 3.4 mm thick) were prepared by using a sugar coater to coat each uncoated tablet with approximately 40 g of sugar coating layer (0.3 mg of pullulan, 1.4 mg of precipitated calcium carbonate, 5.7 mg of talc, 3.9 mg of titanium dioxide, 0.6 mg of polyvinyl pyrrolidone, 1.0 mg of corn starch, 0.2 mg of macrogol 6000, 0.7 mg of hydrous silicon dioxide, and 26 mg of sucrose). These samples were marked with a center line passing the center point of each sugar-coated tablet and extending from one side of the tablet to the other, using the laser and the irradiation conditions described in Table 4. As a result, the tablets changed in color and were marked with a center line with slightly inferior visibility.

### (Example 16) Film-coated Tablets

Film-coated tablets, each having curved surfaces and a cylindrical part, were prepared by coating each uncoated tablet with a coating layer (principal component: hydroxyl propylmethylcellulose terephthalate [Japanese Pharmacopeia]) that contained 3.4 parts by weight of titanium dioxide and 0.4 parts by weight of yellow ferric oxide based on 100 parts by weight of the coating layer, by using an automatic film coating machine. Each tablet was marked with eleven letters in a circular pattern around its curved area, using the laser and under the irradiation conditions described in Table 4. It was confirmed that all the letters were highly identifiable and even the peripheral edge area could be sufficiently marked.

Then, the discolored area irradiated with a laser beam was analyzed in the manner described below. As a sample for analyzing the discolored area, film-coated tablets manufactured under the same conditions as those in Example 13 were used and irradiated with laser, using the laser and under the irradiation conditions described in Table 1. The laser scanning speed for this analysis was 1000 mm/sec.

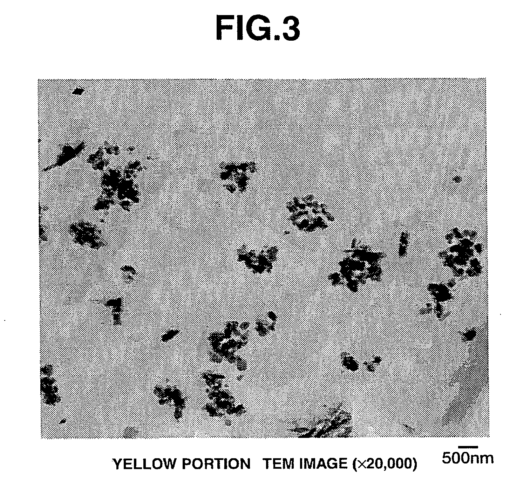

Fig. 3 shows a transmission electron microscope (TEM) photograph showing a yellow area of the film-coated tablet prior to laser irradiation, using a transmission electron microscope (hereinafter simply referred to as "TEM"; JEM-2000FX by JEOL Ltd; acceleration voltage: 200 kV). As shown in Fig. 3, it was observed that titanium dioxide particles shown in black were small. Incidentally, it was confirmed that the black parts were titanium dioxide, from the results of X-ray photoelectron spectroscopy (device: ESCA5400MC [manufactured by PerkinElmer Inc.]; X-ray source: monochromatization Al-Kα ray [15 kV - 500 W]; Pass Energy: 89.45 eV [wide scan], 35.75 eV [high resolution]; resolution [step size]: 1.0 eV [wide scan], 0.1 eV [high resolution]; detection slit: 1.1 mmϕ) and X-ray diffraction (XRD) (device: RAD-C [by RIGAKU Corporation]; X-ray: CuKα ray [using graphite monochromator], 50 kV, 200 mA; measurement: θ-2θ scan, 10≦2θ≦70°; continuous scan, scan speed=4.8°/min).

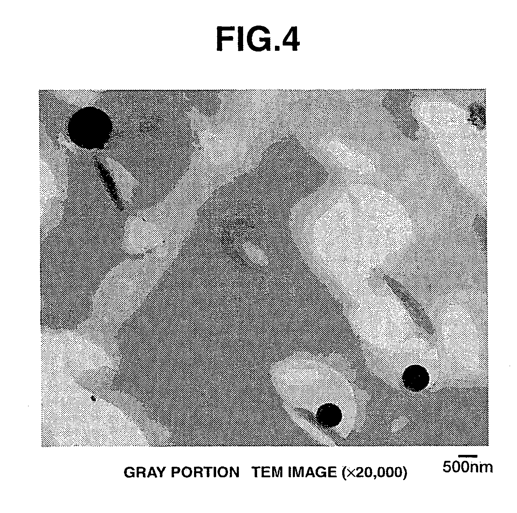

On the other hand, Fig. 4 shows a TEM photograph showing a gray area after laser irradiation. As is apparent from Fig. 4, it was confirmed that the titanium dioxide particles agglomerated, the particle size completely changed from that prior to the laser irradiation, and large particles (shown in black) were formed. Specifically, it was shown that the titanium dioxide agglomerated into spherical particles of about 500 nm after the laser irradiation. It is assumed that the gray color was observed because the particle size after agglomeration was equal to the wavelength of visible light and the agglomerated particles thereby scatter the visible light.

Consequently, it is assumed from the results described above that the particles of titanium dioxide used in the present invention agglomerated and became larger due to laser irradiation and the wavelength of the light reflected on the agglomerated particles changed, thereby causing the laser-irradiated part to change in color. Regarding yellow ferric oxide and red ferric oxide also used in the present invention, it is assumed that the particles of yellow ferric oxide and red ferric oxide agglomerate and become larger due to laser irradiation, and that the wavelength of the light reflected on the agglomerated particles changes, thereby causing the laser-irradiated part to change in color.

### Industrial Applicability

According to the present invention, clear marking can be made at any position on a composition for use in oral administration without being affected so much by the shape and roughness of the surface of the composition, by dispersing at least one type of change in color-inducing oxide selected from the group consisting of titanium dioxide, yellow iron ferric oxide, and red ferric oxide and by irradiating the change in color-inducing oxide with a specific laser light. Therefore, the marking according to the present invention is preferably made on the compositions for use in oral administration with spherical surfaces, such as tablets and capsules, as well as on confectionery with non-level surfaces.

## Claims

1. A method for marking a composition for use in oral administration, comprising the steps of:
dispersing a change in color-inducing oxide in the composition for use in oral administration; and
scanning a surface of the composition for use in oral administration with a laser beam at wavelengths of from 200 nm to 1100 nm and with from 0.1 W to 50 W average power to make a particle of the change in color-inducing oxide agglomerate so as to become discolored.

2. The marking method according to Claim 1, wherein the scanning step is carried out at from 20 mm/sec to 20000 mm/sec.

3. The marking method according to Claim 1 or 2, wherein the scanning step is carried out with an energy per unit area of from 390 mJ/cm² to 21000 mJ/cm².

4. The marking method according to any one of Claims 1 to 3, wherein the laser beam is at least one beam selected from the group consisting of wavelengths of a solid state laser, and wavelengths of second, third, and fourth harmonic waves of the solid laser.

5. The marking method according to any one of Claims 1 to 4, wherein the change in color-inducing oxide is at least one selected from the group consisting of titanium dioxide, yellow ferric oxide, and red ferric oxide.

6. The marking method according to any one of Claims 1 to 5, wherein the composition for use in oral administration has a hardness of from 10 N to 500 N.

7. The marking method according to any one of Claims 1 to 6, wherein the composition for use in oral administration is a molded product.

8. The marking method according to any one of Claims 1 to 6, wherein the composition for use in oral administration comprises a coating layer.

9. The marking method according to Claim 8, wherein the composition comprising the coating layer is a film-coated tablet.

10. The marking method according to any one of Claims 7 to 9, wherein an amount of the titanium dioxide is from 0.01 to 20 parts by weight based on 100 parts by weight of the molded product or the coating layer.

11. The marking method according to any one of claims 7 to 9, wherein an amount of the yellow ferric oxide or the red ferric oxide is from 0.001 to 5 parts by weight based on 100 parts by weight of the molded product or the coating layer.

12. A method for manufacturing a marked composition for use in oral administration, comprising the steps of:
dispersing a change in color-inducing oxide in a composition for use in oral administration; and
scanning a surface of the composition for use in oral administration with a laser beam at wavelengths of from 200 nm to 1100 nm and with from 0.1 W to 50 W average power to make a particle of the change in color-inducing oxide agglomerate so as to become discolored.

13. The manufacturing method according to Claim 12, wherein the scanning step is carried out at from 20 mm/sec to 20000 mm/sec.

14. The manufacturing method according to Claim 12 or 13, wherein the scanning step is carried out with an energy per unit area of from 390 mJ/cm² to 21000 mJ/cm².

15. The manufacturing method according to any one of Claims 12 to 14, wherein the laser beam is at least one beam selected from the group consisting of wavelengths of a solid state laser, and wavelengths of second, third, and fourth harmonic waves of the solid laser.

16. The manufacturing method according to any one of Claims 12 to 15, wherein the change in color-inducing oxide is at least one selected from the group consisting of titanium dioxide, yellow ferric oxide, and red ferric oxide.

17. The manufacturing method according to any one of Claims 12 to 16, wherein the composition for use in oral administration has a hardness of from 10 N to 500 N.

18. The manufacturing method according to any one of Claims 12 to 17, wherein the composition for use in oral administration is a molded product.

19. The manufacturing method according to any one of Claims 12 to 17, wherein the composition for use in oral administration comprises a coating layer.

20. The manufacturing method according to Claim 19, wherein the composition comprising the coating layer is a film-coated tablet.

21. The manufacturing method according to any one of Claims 18 to 20, wherein an amount of the titanium dioxide is from 0.01 to 20 parts by weight based on 100 parts by weight of the molded product or the coating layer.

22. The manufacturing method according to any one of Claims 18 to 20, wherein the amount of the yellow ferric oxide or the red ferric oxide is from 0.001 to 5 parts by weight based on 100 parts by weight of the molded product or the coating layer.

23. A marked composition for oral composition manufactured by the manufacturing method according to any one of Claims 12 to 22.

24. The oral composition according to Claim 23, wherein the composition for use in oral administration is a tablet or a capsule.
